Europäisches Patentamt

European Patent Office

Office européen des brevets

(11) Veröffentlichungsnummer: **0 236 790**
**B1**

(12) **EUROPÄISCHE PATENTSCHRIFT**

(45) Veröffentlichungstag der Patentschrift:
17.10.90

(21) Anmeldenummer: 87102154.9

(22) Anmeldetag: 16.02.87

(51) Int. Cl.⁵: **A61B 6/14**, A61B 6/06,
G21K 1/04

(54) Zahnärztliches Röntgendiagnostikgerät zur Erstellung von Panorama-Schichtaufnahmen vom Kiefer eines Patienten.

(30) Priorität: 28.02.86 DE 3606654

(43) Veröffentlichungstag der Anmeldung:
16.09.87 Patentblatt 87/38

(45) Bekanntmachung des Hinweises auf die Patenterteilung:
17.10.90 Patentblatt 90/42

(84) Benannte Vertragsstaaten:
DE FR GB IT

(56) Entgegenhaltungen:
EP-A- 0 215 757
DE-A- 2 949 946
DE-A- 3 246 114
FR-A- 2 243 672
FR-A- 2 429 584
US-A- 3 849 649
US-A- 4 195 229
US-A- 4 221 971

(73) Patentinhaber: Siemens Aktiengesellschaft,
Wittelsbacherplatz 2, D-8000 München 2(DE)

(72) Erfinder: Döbert, Michael, Zedernstr. 2,
D-6143 Lorsch(DE)
Erfinder: Günther, Werner, Odenwaldstrasse 20,
D-6140 Bensheim(DE)
Erfinder: Heubeck, Erich, Blütenweg 11,
D-6140 Bensheim(DE)

**Beschreibung**

In der zahnärztlichen Röntgentechnik ist es erwünscht, unterschiedliche Panorama-Schichtaufnahmen vom Kiefer eines Patienten erstellen zu können, z.B. Aufnahmen vom gesamten Ober- und Unterkiefer, Aufnahmen nur vom Ober- oder nur vom Unterkiefer, Aufnahmen nur von einem kleinen Kiefer (Kind), oder sogenannte Fernaufnahmen.

Um solche unterschiedlichen Aufnahmen mit einem bekannten Röntgendiagnostikgerät (Prospekt M/D 80/1361 ORTHOPANTOMOGRAPH 10) erstellen zu können, ist es notwendig, die vorher montierte Blende gegen eine andere mit einer anderen Blendenöffnung auszuwechseln. Ein solches Auswechseln ist vergleichsweise umständlich und bei einigen anderen, auf dem Markt befindlichen Geräten relativ aufwendig, da dort mehrere Teile abmontiert und nach Auswechseln und Justieren des Blendeneinsatzes wieder zusammengesetzt werden müssen.

Das Auswechseln der Blenden entfällt zwar bei einem anderen bekannten Röntgendiagnostikgerät, welches durch die Druckschrift EP-A 3-0 215 757 in bezug auf Art. 54 (3) und (4) EPÜ zum Stand der Technik gehört, insofern, als bei diesem Gerät ein Mehrfach-Blendenteil mit einer Vielzahl von unterschiedlichen Blendenöffnungen vorgesehen ist. Das Mehrfach-Blendenteil ist um eine Achslagerung derart schwenkbar gelagert, daß sich die Blendenöffnungen auf den Zentralstrahl der Strahlenquellen ausrichten lassen.

Nachteilig bei diesem bekannten Röntgendiagnostikgerät ist jedoch, daß sich weder das Mehrfach-Blendenteil als solches, noch die einzelnen Blendenöffnungen in bezug auf den Zentralstrahl verstellen lassen, was letztlich bedeutet, daß die Blendenöffnungen nicht exakt auf Strahlenquelle und Film bzw. Filmkassette ausgerichtet werden können.

Der im Anspruch 1 angegebenen Erfindung liegt die Aufgabe zugrunde, demgegenüber eine Verbesserung zu schaffen und ein Röntgendiagnostikgerät anzugeben, mit dem ein exaktes Ausrichten der Blendenöffnungen des Mehrfach-Blendenteiles möglich ist.

Das Mehrfachblendenteil kann in Form eines einteiligen Blendenkörpers mit unterschiedlichen Blendenöffnungen ausgebildet sein oder auch durch mit verschiedenen Blendenöffnungen versehene Einzelblenden gebildet werden, die an einem Träger vorzugsweise auswechselbar gehalten sind. Die Verstellung des Mehrfachblendenteiles, um die jeweilige Blende bzw. Blendenöffnung in die richtige Aufnahmeposition zu bringen, kann von Hand oder auch motorisch erfolgen. Im Falle einer motorischen Verstellung ist es vorteilhaft, die Verstellung durch ein Programm zu steuern. Besondere Vorteile werden in Verbindung mit einer Änderung der Geschwindigkeit der Filmkassette erzielt, weil man damit Aufnahmen nicht nur an verschiedenen Stellen des Kiefers bzw. des Patientenkopfes, sondern auch in unterschiedlichen Schichtlagen erstellen kann.

Weitere vorteilhafte Ausgestaltungen und Weiterbildungen der Erfindung sind in den Unteransprüchen enthalten und der nachfolgenden Beschreibung von zwei Ausführungsbeispielen zu entnehmen.
Es zeigen:

Figur 1 ein Röntgendiagnostikgerät der erfindungsgemäßen Gattung in schaubildlicher Darstellung,

Figur 2 den Röntgenstrahler teilweise aufgeschnitten in schaubildlicher Darstellung,

Figur 3 eine Ausführungsform des Mehrfachblendenteils in schaubildlicher Darstellung,

Figur 4 einen Teil des Mehrfachblendenteils in Frontansicht,

Figur 5 das in Figur 4 gezeigte Mehrfachblendenteil im Schnitt entlang der Linie V/V in Figur 4,

Figur 6 eine andere Ausführungsform eines Mehrfachblendenteils in schaubildlicher Darstellung,

Figur 7 die in Figur 6 gezeigte Zusatzblende in schaubildlicher Darstellung,

Figur 8 das in Figur 6 dargestellte Mehrfachblendenteil im Querschnitt entlang der Linie VIII/VIII in Figur 6,

Figur 9 ein Prinzipschaltbild zur Steuerung des Mehrfachblendenteils,

Figur 10 eine weitere Ausführungsform eines Mehrfachblendenteils in schaubildlicher Darstellung.

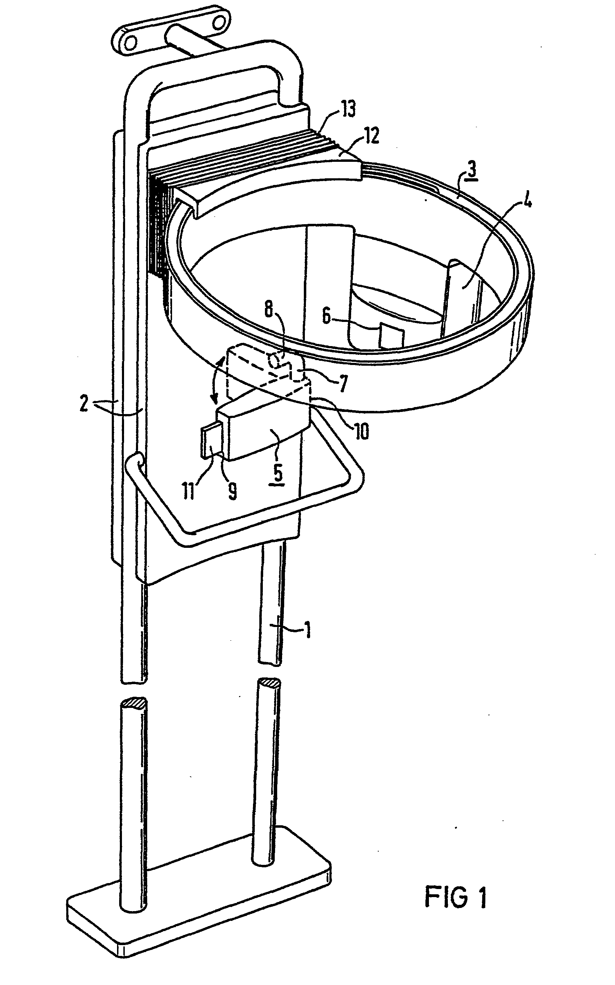

Die Figur 1 zeigt in einer schaubildlichen Darstellung eine mögliche Ausführungsform eines zahnärztlichen Röntgendiagnostikgerätes. Das Gerät enthält ein aus zwei Standrohren gebildetes Stativ 1, an dem ein Laufwagen 2 höhenverstellbar gehalten ist. Am Laufwagen 2 ist eine allgemein mit 3 bezeichnete Dreheinheit in Form eines geschlossenen Ringes gehalten, die einerseits einen Röntgenstrahler 4 und diametral dazu eine Filmkassettenhalterung 5 enthält. Während der Röntgenstrahler 4, dessen gehäuseseitige Strahlenaustrittsöffnung mit 6 bezeichnet ist, am Drehring 3 drehfest angeordnet ist, ist der Filmkassettenhalter 5 mittels vertikaler Achslagerung 7 am abgewinkelten Tragarm 8 in Pfeilrichtung schwenkbar. Der Filmkassettenhalter 5 kann so aus der gestrichelt eingezeichneten Gebrauchsstellung in die mit durchgehenden Linien gezeichnete Nichtgebrauchsstellung gebracht werden, wodurch einerseits der Helferin das Positionieren des zwischen Strahlenquelle und Filmkassettenhalter anzuordnenden Patientenkopfes erleichtert und andererseits das Erstellen von Fernaufnahmen, sog. Ceph-Aufnahmen, ermöglicht wird, für welche bei bisherigen Geräteausführungen die Röntgenstrahlenquelle gedreht werden mußte.

Der Filmkassettenhalter 5 enthält an beiden Stirnseiten schlitzförmige Ein- und Austrittsöffnungen 9, 10, über die die mit 11 bezeichnete Filmkassette eingeführt bzw. nach der Aufnahme entnommen werden kann. Bei der verwendeten Filmkassette handelt es sich um eine flexible, mit Verstärkerfolie versehene Filmkassette, wie sie im Prinzip für intraorale Aufnahmen verwendet wird. Der Transport der Filmkassette erfolgt in nicht näher dargestellter Weise durch einen im Filmkassettenhalter 5 angeordneten elektromotorischen Antrieb.

Der Drehring 3 ist in einem Lagerteil 12 drehbar und gegenüber dem Laufwagen 2 auch schwenkbar gehalten. Die hierzu erforderliche, nicht näher dargestellte Verstellmechanik befindet sich zwischen Laufwagen 2 und Drehring 3; sie ist durch einen Faltenbalg 13 abgedeckt. Die Verstelleinrichtung ermöglicht es, durch entsprechende Steuerung der nicht dargestellten Verstelleinrichtungen den Drehring und damit die Position der Röntgenstrahlenquelle 4 und Filmkassettenhalter 5 in jede beliebige, für den Umlauf um den Patientenkopf notwendige Position zu bringen. In Verbindung mit der Eigendrehbewegung, die der Drehring 3 auch noch um seine Mittelpunktsachse ausführen kann und der im Kassettenhalter 5 verstellbaren Filmkassette, kann so der Bewegungsablauf der gewünschten Aufnahme entsprechend gesteuert werden.

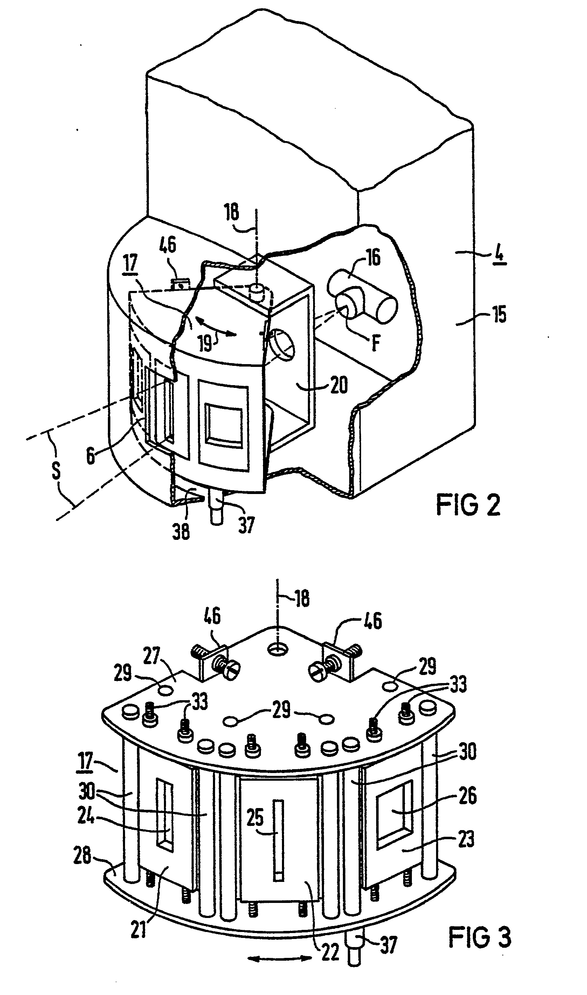

Die Figur 2 zeigt in einer schaubildlichen Darstellung den Röntgenstrahler 4 mit teilweise aufgebrochenem Gehäuse.

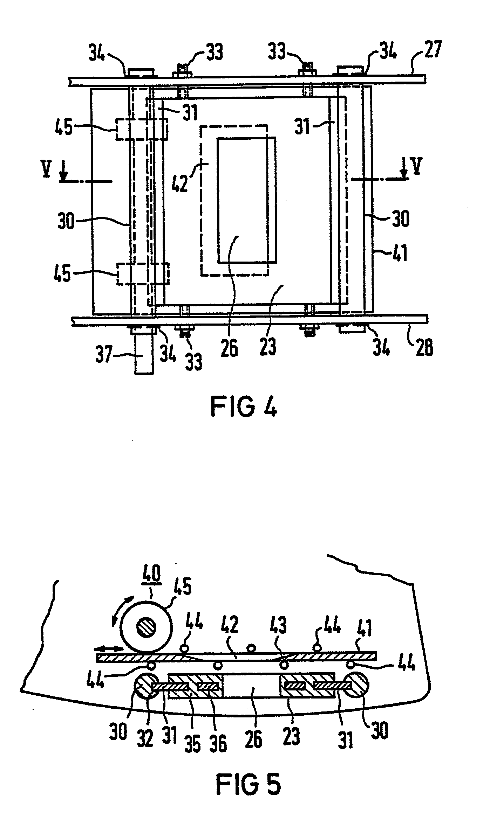

Im rückwärtigen Teil des mit 15 bezeichneten Gehäuses ist in bekannter Weise eine Röntgenröhre 16, also die eigentliche Strahlenquelle, angeordnet, von der aus allgemein mit S bezeichnete Strahlen ausgehen. Der Fokus der Strahlen ist mit F bezeichnet. Im Strahlengang zwischen Strahlenquelle 16 und dem gehäuseseitigen Fenster 6 ist ein Mehrfachblendenteil 17 vorgesehen, welches mittels einer vertikalen Achslagerung 18 in Richtung des angegebenen Pfeiles 19 schwenkbar am Gehäuse 15 des Strahlers 4 gehaltert ist. Zur Lagerung des Mehrfachblendenteils 17 ist ein U-förmig abgewinkeltes, mit einer entsprechenden Öffnung für den Durchtritt der Strahlen versehenes Halteteil 20 vorgesehen. Die Figur 3 zeigt in schaubildlicher Darstellung das Mehrfachblendenteil 17 als Einzelteil. Das Mehrfachblendenteil enthält drei Blendeneinsätze 21, 22 und 23, die jeweils unterschiedlich große Blendenöffnungen 24, 25 und 26 aufweisen. Zur Aufnahme der Blendeneinsätze 21 bis 23 sind zwei etwa die Form eines Kreissektors (von etwa 110 Winkelgraden) aufweisende Platten 27, 28 vorgesehen, die durch mehrere senkrechte Haltebolzen 29 auf Abstand gehalten werden. Die Abstandsbolzen 29 sind zweckmäßigerweise Nietbolzen, die die beiden Platten 27, 28 fest zueinander fixieren.

Zur Halterung der Blendeneinsätze 21 bis 23 sind im peripheren Bereich der Platten 27, 28 paarweise Haltebolzen 30 vorgesehen. Näheres ist aus Figure 4 zu entnehmen, die einen Teil des Mehrfachblendenteils 17, insbesondere die Halterung des Blendeneinsatzes 23, zeigt.

Der Blendeneinsatz 23 (und dies gilt auch für die übrigen Blendeneinsätze) enthält beidseitig Führungsnasen 31, die in Längsschlitze 32 der Haltebolzen 30 eingreifen. Führungsnasen 31 und Schlitze 32 sind so ausgeführt, daß sich die Blendeneinsätze mit geringem Spiel führen lassen.

An den beiden Platten 27, 28 sind Justierschrauben 33 vorgesehen, mit Hilfe derer die Blendeneinsätze auf den Zentralstrahl bzw. auf den Strahlengang des Strahlers 16 ausgerichtet werden können.

Die Halterung der Haltebolzen 30 an den Platten 27, 28 erfolgt durch leicht entfernbare Sicherungsringe 34, wodurch das Auswechseln der Blendeneinsätze sehr leicht vorgenommen werden kann. Hierzu braucht an sich nur ein Sicherungsring eines Haltebolzens entfernt zu werden, danach kann der Haltebolzen nach oben oder unten abgezogen und anschließend der Blendeneinsatz unter leichtem Schwenken und damit Drehen des anderen Haltebolzens herausgenommen werden. Das Einfügen eines mit einer anderen Blendenöffnung versehenen Blendeneinsatzes erfolgt in umgekehrter Weise.

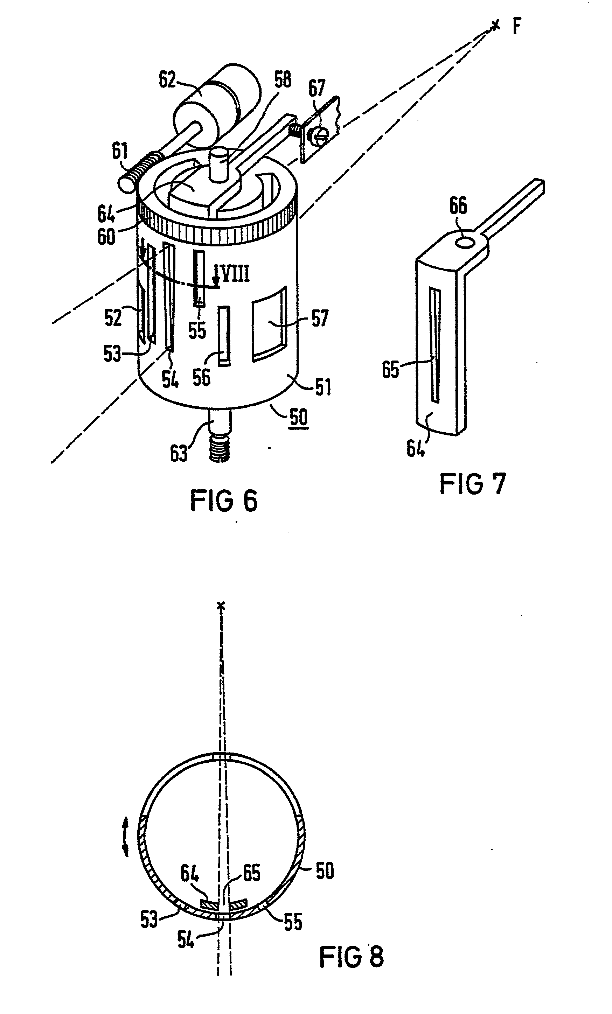

Der Aufbau der Blendeneinsätze wird anhand der Figur 5 und des Blendeneinsatzes 23 erläutert, der speziell für sogenannte Ceph-, also Schädel-Aufnahmen geeignet ist. Der Blendeneinsatz besteht aus einem Grundkörper 35 aus Blei, der die Blendenöffnung 26 beinhaltet und einem darin eingearbeiteten, die Führungsnasen 31 bildenden Blechträger 36.

Wie bereits erwähnt, kann die gesamte Blendenanordnung 17 um die Achslagerung 18 geschwenkt werden. Hierzu ist an der unteren Platte 28 (sieht hierzu Figuren 2 und 3) ein von Hand betätigbarer Stellhebel 37 vorgesehen, der (siehe Figur 2) durch einen entsprechend vorgesehenen Schlitz 38 im Gehäuse 15 nach unten vorsteht und so von Hand ergriffen werden kann. Der Stellhebel 37 ist zweckmäßigerweise gleichsam die Achse eines in Figur 5 mit 40 bezeichneten Verstellantriebes für einen zwischen der Blendenanordnung 17 und dem Fokus (F) zusätzlich vorzusehenden Weichteilfilter 41. Dieser Weichteilfilter 41 besteht vorzugsweise aus einer Kupferplatte mit einem Fenster 42, welches zumindest zu beiden Seiten abgeschrägte Flächen 43 und damit in diesem Bereich unterschiedliche Materialstärke aufweist, wodurch je nachdem, wie weit die Platte mit der schrägen Fensterfläche in den Strahlengang gebracht wird, eine unterschiedliche Weichzeichnung erzielt wird, die speziel bei Ceph-Aufnahmen erwünscht ist. Um eine gute Führung der Kupferplatte 41 zu bekommen, sind auf den beiden Platten 27,28 kleine Führungselemente 44 in Form von Vorsprüngen oder dergleichen vorgesehen.

Der Verstellantrieb kann - wie dargestellt - aus zwei auf der Achse 37 befestigten Reibrädern 45 gebildet sein; anstelle der Reibräder kann auch eine Walze oder ein anderer geeigneter Verstellantrieb, z.B. ein aus einer Verzahnung bestehender Antrieb, vorgesehen sein. Der Antrieb kann ferner, wie anhand des nachfolgenden Ausführungsbeispieles beschrieben, auch ein elektromotorischer Antrieb sein, der mit Hilfe einer Steuereinrichtung je nach Wahl der vorzusehenden Aufnahme das Mehrfachblendenteil so weit verstellt, bis die zugehörige Blende bzw. Blendenöffnung sich im Strahlengang des Strahlers befindet.

Der Verstellweg (Schwenkweg) des Mehrfachblendenteils ist durch beidseitig an der oberen Platte 27 angeordnete Endanschlagglieder 46 (Figuren 2 und 3) begrenzt. Diese sind, um die Blendenanordnung genau auf den Strahlengang einstellen zu können, einstellbar ausgebildet. Um ferner eine eindeutige Zuordnung und Fixierung der Einzelblenden auf den Strahlengang zu bekommen, kann die Mittenstellung, in der die Blendenöffnungen genau im Strahlengang liegen, durch geeignete, in der Zeichn-

nung nicht dargestellte Rastmittel, z.B. durch eine Kugelrastung, festgelegt sein.

Die Figuren 6 bis 8 zeigen eine weitere Ausführungsform eines Mehrfachblendenteils. Im Gegensatz zu dem zuvor beschriebenen Mehrfachblendenteil 17 ist das in Figur 6 in schaubildlicher Darstellung aufgezeigte Mehrfachblendenteil 50 als einteiliger hohlzylindrischer Blendenkörper ausgebildet, an dessen zylindrischem Mantel 51 eine Strahlendurchtrittsöffnung 52 sowie Blendenöffnungen 53 bis 57 mit unterschiedlicher Blendenweite und -höhe vorgesehen sind. Die Blendenöffnungen 53 bis 57 sind für folgende Aufnahmen geeignet:
Blendenöffnung 53Unter- und Oberkiefer von Kindern
Blendenöffnung 54Unter- und Oberkiefer von Erwachsenen (Standard-Aufnahmen)
Blendenöffnung 55nur Oberkiefer
Blendenöffnung 56nur Unterkiefer
Blendenöffnung 57Ceph (Schädel)

Die Öffnung 52 ist dazu vorgesehen, um bei eingestellter Blendenöffnung 57 einen freien Strahlendurchgang zu haben.

Der rotationssymmetrisch ausgebildete Blendenkörper 50 ist um seine Rotationsachse 58 schwenkbar ausgebildet. Die Schwenkachse 58 entspricht der Schwenkachse 18, um die(Figur 2) das Mehrfachblendenteil 17 schwenkbar ist.

Die Verstellung des Blendenkörpers 50 erfolgt elektromotorisch, wozu am Umfang des Blendenkörpers 50 eine Verzahnung 60 vorgesehen ist, in die ein Ritzel 61 eines elektromotorischen Antriebs 62 eingreift. Als Antrieb kann ein Schrittmotor oder ein anderer geeigneter Antrieb vorgesehen sein.

Um das Mehrfachblendenteil 50 in horizontaler Richtung auf den Strahlengang ausrichten zu können, ist an geeigneter Stelle eine Stellschraube 63 vorgesehen.

Um insbesondere bei Standard-Aufnahmen im Frontzahnbereich eine abgeschwächte Strahlungsintensität zu bekommen, ist im Strahlengang zwischen dem Fokus (F) des Strahlers 16 und dem Strahlenaustritt am Blendenkörper 51 eine Zusatzblende 64 (Bleiblende) vorgesehen, die in Figur 7 als Einzelteil in schaubildlicher Darstellung aufgezeigt ist. Die Zusatzblende 64 enthält eine der Blendenöffnung 54 entsprechende Blendenöffnung 65 und ist über eine Bohrung 66 an der Schwenkachse 58 schwenkbar gehalten. Die Blendenöffnung kann so, wie in Figur 8 im Prinzip dargestellt, gegenüber der Blendenöffnung 54 des Blendenkörpers 50 etwas versetzt eingestellt werden, wodurch sich eine Kantenverschiebung und damit eine unterschiedliche Strahlungsintensität auf dem Film innerhalb der Schlitzbreite ergibt. Das Maß der Kantenverschiebung kann mittels einer Stellschraube 67 stufenlos variiert werden.

Ein weiterer wesentlicher Vorteil des erfindungsgemäß vorgesehenen Mehrfachblendenteils ist, daß man unter Beibehaltung der sonstigen Konstruktion auf relativ einfache Weise eine Anpassung an länderspezifische Formate mit anderen Maßeinheiten (z.B. Zollmaß) erreichen kann, indem man entweder die einzelnen Blendeneinsätze oder den gesamten Blendenkörper austauscht.

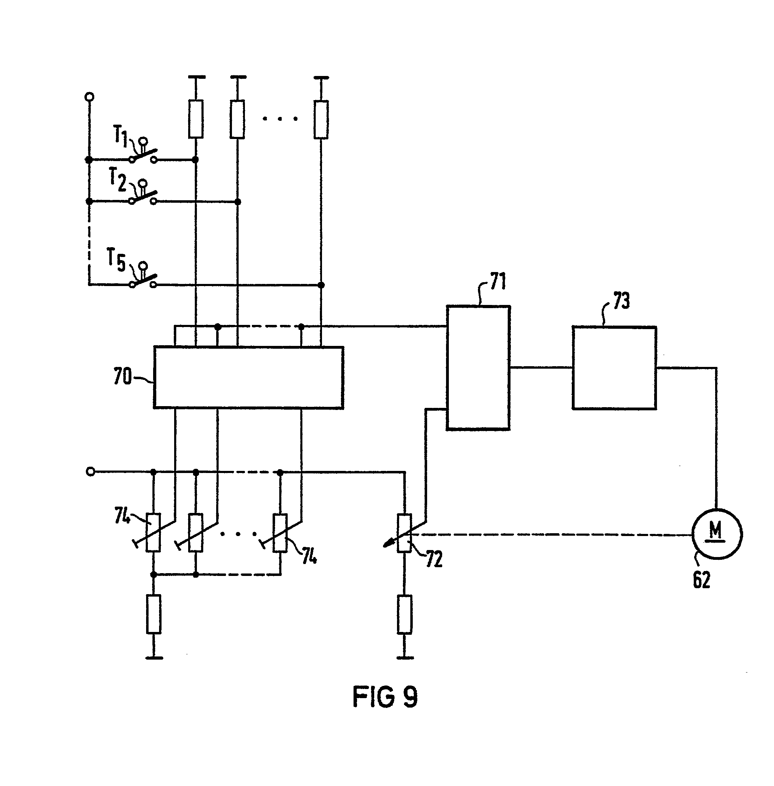

Die Figur 9 zeigt anhand eines Prinzipschaltbildes eine Möglichkeit der Steuerung des Antriebsmotors 62. Es wird davon ausgegangen, daß jede Blendenposition durch einen bestimmten Sollwert festgelegt ist, der in einem geeigneten Speicher 70, z.B. einem ROM-Speicher, abgelegt ist. Der betreffende Speicherwert kann mittels Tasten T1 bis T6 (entsprechend der anwählbaren Blendenöffnungen 52 bis 57) abgerufen und einem Soll-/Ist-Vergleicher 71 zugeführt werden, dem über einen Istwertgeber 72 die jeweilige Istposition des Motors 62 und damit des Blendenkörpers 50 zugeführt wird. Ein Steuerungsbaustein 73 treibt den Antriebsmotor 62 so lange an, bis Soll- und Istwerte übereinstimmen, d.h. bis die abgerufene Blende bzw. Blendenöffnung sich in der vorgesehenen Aufnahmeposition befindet. Zur genauen Justierung der Blendenposition sind ferner noch diverse Einstellpotentiometer 74 vorgesehen.

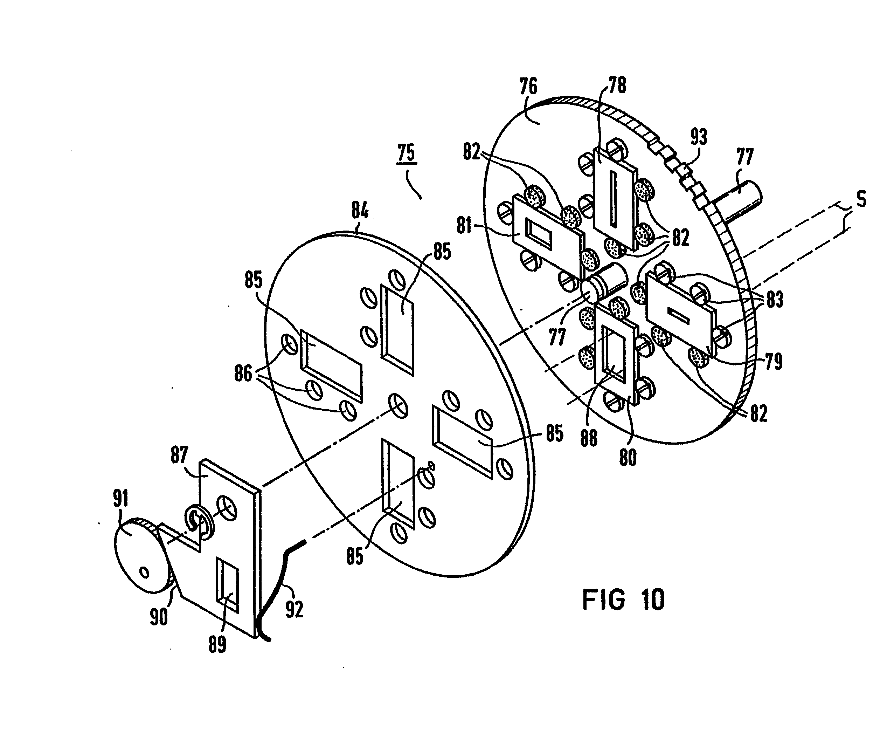

Die Figur 10 zeigt in einer schaubildlichen Darstellung eine weitere Ausführungsform eines Mehrfachblendenteils. Das allgemein mit 75 bezeichnete Mehrfachblendenteil enthält ein scheibenförmiges Trägerteil 76, welches mittels einer Achse 77 im Gehäuse des Röntgenstrahlers 4 drehbar gelagert ist. Hierzu kann ein dem Halteteil 20 in Figur 2 adäquates Halteteil vorgesehen sein. Das Trägerteil 76 ist so gehaltert, daß sich durch Drehen die Blendenöffnungen von vier am Trägerteil 76 in einer senkrecht zur Drehachse liegenden Ebene angeordneten Blendeneinsätzen 78 bis 81 auf den Strahlengang S des Röntgenstrahlers 16 ausrichten lassen. Die Blendeneinsätze 78 bis 81 sind zum genauen Justieren ihrer Blendenöffnungen verstellbar angeordnet. Im vorliegenden Ausführungsbeispiel liegen an zwei benachbarten Seitenkanten der Blendeneinsätze jeweils Federelemente in Form von Schaumstoffkörpern 82 und an den verbleibenden beiden anderen Seitenkanten exzentrisch gelagerte Stellschrauben 83 an. Blendeneinsätze, Federelemente und Exzenterstellschrauben sind zweckmäßigerweise in entsprechenden, hier nicht gezeigten Vertiefungen des Trägerteils 76 eingelegt, so daß ihre Außenkanten bündig mit der Stirnfläche des Trägerteils 76 abschließen können. Auf dieser Stirnfläche liegt im montierten Zustand eine ebenfalls scheibenförmige Abdeckplatte 84 an, die mittels nicht dargestellter Befestigungselemente mit dem Trägerteil 76 verbunden ist. Die Abdeckplatte 84 weist gleich große, rechteckige Öffnungen 85 auf, die zumindest der größten Blendenöffnung der Blendeneinsätze 78 bis 81 entsprechen. Ferner sind der Größe der Exzenterstellschrauben 83 entsprechende Bohrungen 86 vorhanden, durch die hindurch nach Montage der beiden Teile 76 und 84 ein Werkzeug zur Verstellung der Exzenterschrauben 83 geführt werden kann. Auf der Achse 77 ist schließlich noch ein dem Weichteilfilter 41 in Fig. 4 und 5 entsprechendes Weichteilfilter 87 aufgesetzt, welches eine der Blendenöffnung eines Blendeneinsatzes, beispielsweise der Öffnung 88 des Blendeneinsatzes 80 entsprechende Öffnung 89 aufweist. Das Weichteilfilter 87 besteht, wie das Weichteilfilter 41, aus einem Material (z.B. Kupfer) mit dem sich eine unterschiedliche Weichteilzeichnung des Objekts, z.B. bei Schädelaufnahmen, erzielen läßt. Zur Erzielung eines weichen Überganges können auch hier schräge Fensterflächen vorgesehen sein. Das Weichteilfilter 87 enthält eine schräge Fläche 90, an die ein exzentrisch gelagertes Stellrad 91 anliegt, mit welchem das Weichteilfilter 87, entgegen der Kraft einer auf der gegenüberliegenden Seitenkante Weichteilfilteröffnung 89 mehr oder weniger in den Strahlengang des Röntgenstrahlers einschwenkbar ist.

Sowohl das Stellrad 91 als auch das Trägerteil 76 können entweder von Hand oder motorisch verstellt werden, wozu zweckmäßigerweise am Umfang eine entsprechende Profilierung vorgesehen ist, die für das Trägerteil 76 mit 93 bezeichnet ist. Diese Profilierung kann beispielsweise bei motorischer Verstellung mit Riemenantrieb mit einem im Profil entsprechenden Riemen zusammenwirken.

Die Blendeneinsätze 78 bis 81 sind in ihren Außenabmessungen gleich, so daß sie untereinander leicht ausgetauscht werden können bzw. daß die Reihenfolge ihrer Anordnung auf dem Trägerteil 76 beliebig wählbar ist. Demgemäß sind auch die Öffnungen 85 in der Abdeckplatte und die (nicht dargestellten) Öffnungen im Trägerteil 76 gleich groß.

Anstelle der gezeigten scheibenförmigen Schaumstoffplättchen können selbstverständlich auch anders gestaltete Federelemente vorgesehen werden.

**Patentansprüche**

1. Zahnärztliches Röntgendiagnostikgerät zur Erstellung von Panorama-Schichtaufnahmen vom Kiefer eines Patienten, enthaltend eine zwischen Strahlenquelle (4) und Filmkassette (5) einzubringende Blende zur Begrenzung des Strahlenbündels in Form eines Mehrfachblendenteiles (17, 50, 75) mit einer Vielzahl von unterschiedlichen Blendenöffnungen (24 bis 26; 53 bis 57, 88), wobei das Mehrfachblendenteil um eine Achslagerung (18, 58, 70) derart schwenkbar gehalten ist, daß sich die Blendenöffnungen (24 bis 26; 53 bis 57, 88) jeweils auf den Strahlengang (Zentralstrahl) der Strahlenquelle (4) aufrichten lassen, sowie Justiermittel (33, 63, 82, 83), mit deren Hilfe die Blendenöffnungen (24 bis 26, 53 bis 57, 88) auf den Strahlengang (Zentralstrahl) der Strahlenquelle ausgerichtet werden können.

2. Zahnärztliches Röntgendiagnostikgerät nach Anspruch 1, **dadurch gekennzeichnet**, daß das Mehrfachblendenteil als einteiliger Blendenkörper (50) mit unterschiedlichen Blendenöffnungen (53 bis 57) ausgebildet ist.

3. Zahnärztliches Röntgendiagnostikgerät nach Anspruch 1, **dadurch gekennzeichnet**, daß das Mehrfachblendenteil einen rotationssymmetrisch ausgebildeten Blendenkörper (50, 76) enthält, der um seine Rotationsachse (58, 77) schwenkbar angeordnet ist.

4. Zahnärztliches Röntgendiagnostikgerät nach Anspruch 3, **dadurch gekennzeichnet**, daß ein zylindrischer Blendenkörper (50) vorgesehen ist, an dessen Mantelfläche (51) die unterschiedlichen Blendenöffnungen (53 bis 57) vorgesehen sind.

5. Zahnärztliches Röntgendiagnostikgerät nach Anspruch 3, **dadurch gekennzeichnet**, daß am Umfang des Blendenkörpers (50, 75) eine mit einem motorischen Antrieb (62) zusammenwirkende Verzahnung (60, 93) angeordnet ist.

6. Zahnärztliches Röntgendiagnostikgerät nach Anspruch 3, **dadurch gekennzeichnet**, daß an der Achslagerung (58) des Blendenkörpers (50) eine Zusatzblende (64) schwenkbar angeordnet ist, deren Blendenöffnung (65) so gewählt ist, daß bei Verstellung der Zusatzblende (64) die wirksame Blendenweite (Spaltbreite) der Blendenöffnung (54) des Blendenkörpers (50) verändert werden kann.

7. Zahnärztliches Röntgendiagnostikgerät nach Anspruch 6, **dadurch gekennzeichnet**, daß der Verstellbereich der Zusatzblende (64) durch veränderbare Anschlagmittel (67) einstellbar ist.

8. Zahnärztliches Röntgendiagnostikgerät nach Anspruch 1, **dadurch gekennzeichnet**, daß das Mehrfachblendenteil ein um die Schwenkachse (18, 77) schwenkbares Trägerteil (27 bis 29, 76) enthält, an dem mehrere, die unterschiedlichen Blendenöffnungen (24 bis 26, 88) enthaltende Blendeneinsätze (21 bis 23, 78 bis 81) auswechselbar gehalten sind.

9. Zahnärztliches Röntgendiagnostikgerät nach Anspruch 8, **dadurch gekennzeichnet**, daß als Trägerteil zwei in vertikaler Richtung beabstandete, etwa einen Sektor eines Kreises bildende Platten (27, 28) vorgesehen sind, an deren peripheren Enden mit Längsschlitzen (32) versehene Haltebolzen (30) angeordnet sind, in deren Schlitze (32) die mit dazu passenden Führungselementen (31) versehenen Einzelblenden (21 bis 23) einführbar sind und die Haltebolzen (30) mittels leicht lösbarer Befestigungselemente (34) an den Platten (27, 28) befestigt sind.

10. Zahnärztliches Röntgendiagnostikgerät nach Anspruch 1, dadurch gekennzeichnet, daß ein Mehrfachblendenteil (75) mit einem vorzugsweise scheibenförmigen Trägerteil (76) für die unterschiedlichen Blendenöffnungen (88) enthaltende Blendeneinsätze (78 bis 81) vorgesehen ist, welche in einer Ebene senkrecht zur Achslagerung (77) so angeordnet sind, daß ihre Blendenöffnungen durch Drehen des Trägerteils (76) auf den Strahlengang (S) der Strahlenquelle (16) ausrichtbar sind.

11. Zahnärztliches Röntgendiagnostikgerät nach Anspruch 1, dadurch gekennzeichnet, daß das Mehrfachblendenteil (50, 75) Blendeneinsätze (21 bis 23; 78 bis 81) enthält, an denen die Justiermittel (33, 82, 83) angeordnet sind.

12. Zahnärztliches Röntgendiagnostikgerät nach Anspruch 10, dadurch gekennzeichnet, daß die Blendeneinsätze (78 bis 81) zwischen einerseits einem scheibenförmigen Trägerteil (76) und andererseits einer ebenfalls scheibenförmigen, mit dem Träger verbundenen Abdeckplatte (84) eingelegt und mit Hilfe von an zwei Seitenkanten anliegenden, von außen zugänglichen Verstellgliedern (83) und an den beiden anderen Seitenkanten angreifenden Federelementen (82) in Ebene ihrer Anordnung verstellbar sind.

13. Zahnärztliches Röntgendiagnostikgerät nach Anspruch 10, **dadurch gekennzeichnet**, daß in ihrer Baugröße gleiche Blendeneinsätze (78 bis 81) vorgesehen sind.

14. Zahnärztliches Röntgendiagnostikgerät nach Anspruch 8, **dadurch gekennzeichnet**, daß die Blendeneinsätze (21 bis 23; 78 bis 81) aus einem die Blendenöffnung (24 bis 26, 88) enthaltenden Grundkörper (35) aus Blei und einem dazwischen angeordneten, die Führungselemente (31) enthaltenden Blechträger (36) bestehen.

15. Zahnärztliches Röntgendiagnostikgerät nach Anspruch 1, **dadurch gekennzeichnet**, daß wenigstens einer Blende (23, 80) ein Weichteilfilter (41, 87) zugeordnet ist, welches am Mehrfachblendenteil (17, 50, 75) verstellbar gehalten ist.

16. Zahnärztliches Röntgendiagnostikgerät nach Anspruch 10 und 15, **dadurch gekennzeichnet** daß das Weichteilfilter (87) an der Drehachse (77) des Trägerteils (76) mittels einer Stelleinrichtung (90, 91) derart schwenkbar gehalten ist, daß die Lage der Weichteilfilteröffnung (89) relativ zu der im Strahlengang befindlichen Blendenöffnung (88) veränderbar ist.

17. Zahnärztliches Röntgendiagnostikgerät nach Anspruch 15, **dadurch gekennzeichnet**, daß als Verstellantrieb für das Weichteilfilter (41, 87) ein Reiboder Zahnradantrieb (40, 91) vorgesehen ist, welches von Hand oder motorisch antreibbar ist.

18. Zahnärztliches Röntgendiagnostikgerät nach Anspruch 17, **dadurch gekennzeichnet**, daß die Achse des Verstellantriebes (40) für die Weichteilfilterplatte (41) gleichsam Verstellachse für das Mehrfachblendenteil (17, 50) ist.

19. Zahnärztliches Röntgendiagnostikgerät nach Anspruch 1, **dadurch gekennzeichnet**, daß die Schwenkachse (18, 58, 77) des Mehrfachblendenteils (17, 50, 75) nahe dem Brennpunkt (F) des Strahlers (4), vorzugsweise im Brennpunkt selbst, liegt.

## Claims

1. A dental x-ray diagnostic apparatus for creating panorama planigraphic exposures of the jaw of a patient, containing a diaphragm to be introduced between the radiation source (4) and film cassette (5), for defining the beam of rays, the diaphragm being in the form of a multiple diaphragm part (17, 50, 75) with a plurality of different diaphragm apertures (24 to 26; 53 to 57, 88), wherein the multiple diaphragm part is pivoted around an axle bearing (18, 58, 70) in such a way that the diaphragm apertures (24 to 26; 53 to 57, 88) can be aligned in each case relative to the beam path (central ray) of the radiation source (4), as well as adjusting means (33, 63, 82, 83) with the aid of which the diaphragm apertures (24 to 26, 53 to 57, 88) can be aligned with the beam path (central ray) of the radiation source.

2. A dental x-ray diagnostic apparatus according to claim 1, characterised in that the multiple diaphragm part is formed as a one piece diaphragm body (50) with varying diaphragm apertures (53 to 57).

3. A dental x-ray diagnostic apparatus according to claim 1, characterised in that the multiple diaphragm part contains a rotationally symmetrically constructed diaphragm body (50, 76), which is pivotably arranged about its rotational axis (58, 77).

4. A dental x-ray diagnostic apparatus according to claim 3, characterised in that a cylindrical diaphragm body (50) is provided, on the generated surface (51) of which the varying diaphragm apertures (53 to 57) are provided.

5. A dental x-ray diagnostic apparatus according to claim 3, characterised in that arranged on the periphery of the diaphragm body (50, 75) there is a gearing (60, 93) cooperating with a driving mechanism (62).

6. A dental x-ray diagnostic apparatus according to claim 3, characterised in that on the axle bearing (58) of the diaphragm body (50) there is pivotably arranged an additional diaphragm (64), the diaphragm aperture (65) of which is selected such that when adjusting the additional diaphragm (64) the effective diaphragm width (width of gap) of the diaphragm aperture (54) of the diaphragm body (50) can be altered.

7. A dental x-ray diagnostic apparatus according to claim 6, characterised in that the range of adjustment of the additional diaphragm (64) is able to be set by variable stopping means (67).

8. A dental x-ray diagnostic apparatus according to claim 1, characterised in that the multiple diaphragm part contains a support part (27 to 29, 76) pivotable about the pivoting axis (18, 77), on which several diaphragm inserts (21 to 23, 78 to 81) containing the various diaphragm apertures (24 to 26, 88) are held so that they can be interchanged.

9. A dental x-ray diagnostic apparatus according to claim 8, characterised in that, as support part, there are provided two plates (27, 28), spaced apart, standing in the vertical direction, forming an approximate sector of a circle, on the peripheral ends of which plates there are arranged retaining pins (30) provided with longitudinal slots (32), in which slots (32) the individual diaphragms (21 to 23), provided with guiding elements (31) adapted thereto, are able to be inserted, and the retaining pins (30) are secured on the plates (27, 28) by means of easily releasable securing elements (34).

10. A dental x-ray diagnostic apparatus according to claim 1, characterised in that a multiple diaphragm part (75) is provided with a preferably discshaped support part (76) for the diaphragm inserts (78 to 81) containing various diaphragm apertures (88), which inserts are arranged in a plane perpendicular to the axle bearing (77), so that their diaphragm apertures are able to be aligned into the beam path (S) of the radiation source (16) by rotating the support body (76).

11. A dental x-ray diagnostic apparatus according to claim 1, characterised in that the multiple diaphragm part (50, 75) contains diaphragm inserts (21 to 23; 78 to 81), on which the adjusting means (33, 82, 83) are arranged.

12. A dental x-ray diagnostic apparatus according to claim 10, characterised in that the diaphragm inserts (78 to 81) are laid between, on the one hand, a disc-shaped support part (76) and, on the other hand, a cover plate (84), also disc-shaped, and connected to the support, and with the aid of adjustment members (83) resting on two side edges, accessible from outside, and spring elements (82) engaging on the two other side edges, are adjustable in the plane of their arrangement.

13. A dental x-ray diagnostic apparatus according to claim 10, characterised in that diaphragm inserts (78 to 81) are provided which are of like overall size.

14. A dental x-ray diagnostic apparatus according to claim 8, characterised in that the diaphragm inserts (21 to 23; 78 to 81) consist of a basic body (35) of lead containing the diaphragm aperture (24 to 26, 88) and a plate support (36), arranged therebetween, and containing the guiding elements (31).

15. A dental x-ray diagnostic apparatus according to claim 1, characterised in that allocated to at least one diaphragm (23, 80) there is a soft part filter (41, 87), which is adjustably mounted on the multiple diaphragm part (17, 50, 75).

16. A dental x-ray diagnostic apparatus according to claims 10 and 15, characterised in that the soft part filter (87) is pivotably mounted on the rotational axis (77) of the support part (76) by means of an adjusting device (90, 91), in that the position of the soft part filter opening (89) is variable relative to the diaphragm aperture (88) located in the beam path.

17. A dental x-ray diagnostic apparatus according to claim 15, characterised in that, as the adjusting drive for the soft part filter (41, 87), there is provided a friction or toothed wheel drive (40, 91), which is able to be driven manually or by motor.

18. A dental x-ray diagnostic apparatus according to claim 17, characterised in that the axle of the adjusting drive (40) for the soft part filter plate (41) is at the same time the adjusting axle for the multiple diaphragm part (17, 50).

19. A dental x-ray diagnostic apparatus according to claim 1, characterised in that the pivoting axis (18, 58, 77) of the multiple diaphragm part (17, 50, 75) lies close to the focal point (F) of the radiation source (4), preferably at the focal point itself.

**Revendications**

1. Appareil de radiodiagnostic dentaire servant à réaliser des tomographies panoramiques de la mâchoire d'un patient, contenant un diaphragme devant être inséré entre une source de rayonnement (4) et une cassette à film (5) et servant à limiter le faisceau de rayonnement et se présentant sous la forme d'une pièce (17, 50, 75) à diaphragmes multiples comportant une multiplicité d'ouvertures de diaphragmes différentes (24 à 26; 53 à 57, 88), la pièce à diaphragmes multiples étant maintenue de manière à pouvoir tourner autour d'un palier (18, 58, 70) de telle sorte que les ouvertures de diaphragmes (24 à 26; 53 à 57, 88) peuvent être alignées respectivement sur le trajet du rayonnement (rayon central) de la source de rayonnement (4), ainsi que des moyens d'ajustement (33, 63, 82, 83), à l'aide desquels les ouvertures de diaphragmes (24 à 26; 53 à 57, 88) peuvent être alignées sur le trajet du rayonnement (rayon central) de la source de rayonnement.

2. Appareil de radiodiagnostic dentaire suivant la revendication 1, caractérisé par le fait que la pièce à diaphragmes multiples est réalisée sous la forme d'un diaphragme monobloc (5) comportant différentes ouvertures de diaphragmes (53 à 57).

3. Appareil de radiodiagnostic dentaire suivant la revendication 1, caractérisé par le fait que la pièce à diaphragmes multiples contient un corps à diaphragmes (50, 70) à symétrie de révolution, qui est monté de manière à pouvoir pivoter autour de son axe de rotation (58, 77).

4. Appareil de radiodiagnostic dentaire suivant la revendication 3, caractérisé par le fait qu'il est prévu un corps cylindrique à diaphragmes (50), sur la surface enveloppe (51) duquel sont prévues les différentes ouvertures de diaphragmes (53 à 57).

5. Appareil de radiodiagnostic dentaire suivant la revendication 3, caractérisé par le fait que sur le pourtour du corps à diaphragmes (50, 75) est disposée une denture (60, 93), qui coopère avec un dispositif d'entraînement à moteur (62).

6. Appareil de radiodiagnostic dentaire suivant la revendication 3, caractérisé par le fait que sur le palier (58) du corps à diaphragmes (5) est disposé, de manière à pouvoir pivoter, un diaphragme supplémentaire (64), dont l'ouverture (65) est choisie de manière que, lors du réglage du diaphragme supplémentaire (64), la largeur active (largeur de fente) de l'ouverture de diaphragme (54) du corps à diaphragmes (50) peut être modifiée.

7. Appareil de radiodiagnostic dentaire suivant la revendication 6, caractérisé par le fait que la plage de réglage du diaphragme supplémentaire (64) est réglable à l'aide de moyens réglables de butée (67).

8. Appareil de radiodiagnostic dentaire suivant la revendication 1, caractérisé par le fait que la pièce à diaphragmes multiples contient une partie de support (27 à 29, 66), qui peut pivoter autour de l'axe de pivotement (18, 77) et sur laquelle plusieurs inserts de diaphragmes (21 à 23, 78 à 81), qui contiennent les différentes ouvertures de diaphragmes (24 à 26, 88), sont maintenus de manière à être interchangeables.

9. Appareil de radiodiagnostic dentaire suivant la revendication 8, caractérisé par le fait qu'il est prévu, comme partie de support, deux plaques (27, 28), qui sont distantes verticalement, forment approximativement un secteur circulaire et sur les extrémités périphériques desquelles sont disposés des boulons de retenue (30), qui possèdent des fentes longitudinales (32) dans lesquelles peuvent être insérés les diaphragmes individuels (21 à 23) pourvus d'éléments de guidage (31) adaptés à ces fentes, tandis que les boulons de retenue (30) sont fixés aux plaques (27, 28) à l'aide d'éléments de fixation (34) pouvant être aisément desserrés.

10. Appareil de radiodiagnostic dentaire suivant la revendication 1, caractérisé par le fait qu'il est prévu une pièce à diaphragmes multiples (75) comportant une partie de support (76) de préférence en forme de disque, servant à supporter des inserts de diaphragmes (78 à 81), qui comportent différentes ouvertures de diaphragmes (88) et sont disposées dans un plan perpendiculairement au palier (77) de telle sorte que leurs ouvertures peuvent être alignées, par rotation de la partie de support (76), sur le trajet du rayonnement (S) de la source de rayonnement (16).

11. Appareil de radiodiagnostic dentaire suivant la revendication 1, caractérisé par le fait que la pièce à diaphragmes multiples (50, 75) contient des inserts de diaphragmes (21 à 23; 78 à 80), sur lesquels sont disposés les moyens d'ajustement (33, 82, 83).

12. Appareil de radiodiagnostic dentaire suivant la revendication 10, caractérisé par le fait que les inserts de diaphragmes (78 à 80) sont insérés entre d'une part une partie de support en forme de disque (76) et d'autre part une plaque de fermeture (84) également en forme de disque, reliée au support, et que leur disposition peut être réglée dans leur plan d'installation à l'aide d'organes de réglage (83) situés sur deux bords latéraux et accessibles de l'extérieur, et d'éléments de ressorts (82) qui s'accrochent aux deux autres bords latéraux.

13. Appareil de radiodiagnostic dentaire suivant la revendication 10, caractérisé par le fait qu'il est prévu des inserts de diaphragmes (78 à 81) possédant des dimensions hors-tout identiques.

14. Appareil de radiodiagnostic dentaire suivant la revendication 8, caractérisé par le fait que les inserts de diaphragmes (21 à 23; 78 à 81) sont constitués par un corps de base (35) en plomb, contenant l'ouverture de diaphragme (24 à 26, 88), et par un support en tôle (36) disposés dans ce corps et contenant les éléments de guidage (21).

15. Appareil de radiodiagnostic dentaire suivant la revendication 1, caractérisé par le fait qu'à au moins un diaphragme (23, 81) est associé un filtre partiel pour les parties molles (41, 87), qui est maintenu de manière à être réglable sur la pièce à diaphragmes multiples (17, 50, 75).

16. Appareil de radiodiagnostic suivant l'une des revendications 10 et 15, caractérisé par le fait que le filtre partiel pour les parties molles (87) est monté de manière à pouvoir pivoter sur l'axe de rotation (77) de la partie de support (76) à l'aide d'un dispositif de réglage (90, 91), de telle sorte que la position de l'ouverture (89) du filtre partiel pour les parties molles par rapport à l'ouverture de diaphragme (88) située sur le trajet du rayonnement peut être modifié.

17. Appareil de radiodiagnostic dentaire suivant la revendication 1, caractérisé par le fait qu'il est prévu, comme dispositif d'entraînement de réglage pour le filtre partiel pour les parties molles (41, 87), un dispositif d'entraînement par friction ou à pignons (40, 91), qui peut être entraîné manuellement ou à l'aide d'un moteur.

18. Appareil de radiodiagnostic dentaire suivant la revendication 17, caractérisé par le fait que l'axe du dispositif d'entraînement de réglage (40) pour la plaque (41) du filtre partiel pour les parties molles est pour ainsi dire l'axe de réglage pour la pièce à diaphragmes multiples (17, 50).

19. Appareil de radiodiagnostic dentaire suivant la revendication 1, caractérisé par le fait que l'axe de pivotement (18, 58, 77) de la pièce à diaphragmes multiples (17, 50, 75) est proche du foyer (F) de l'émetteur de rayonnement (4) et de préférence est situé au foyer lui-même.

FIG 1

FIG 2

FIG 3

FIG 4

FIG 5

EP 0 236 790 B1

FIG 6

FIG 7

FIG 8

FIG 9

FIG 10

EP 0 236 790 B1